# EUROPÄISCHE PATENTSCHRIFT

(11) **EP 0 701 565 B1**
(45) Veröffentlichungstag und Bekanntmachung des Hinweises auf die Patenterteilung: **28.10.1998**
(21) Anmeldenummer: 94913100.7
(22) Anmeldetag: 31.03.1994
(51) Int. Cl.: C07J 71/00, A61K 31/58

(54) **NEUE PREDNISOLONDERIVATE**
NEW PREDNISOLONE DERIVATES
NOUVEAUX DERIVES DE PREDNISOLONE

(30) Priorität: 02.04.1993 CH 1023/93
(43) Veröffentlichungstag der Anmeldung: 20.03.1996
(73) Patentinhaber: Byk Gulden Lomberg Chemische Fabrik GmbH, 78467 Konstanz (DE)
(72) Erfinder: GUTTERER, Beate, D-78476 Allensbach (DE); AMSCHLER, Hermann, D-78315 Radolfzell (DE); FLOCKERZI, Dieter, D-7867 Allensbach (DE); RIEDEL, Richard, D-88339 Bad Waldsee (DE); POSTIUS, Stefan, D-78467 Konstanz (DE); STOECK, Michael, D-78315 Radolfzell (DE); BEUME, Rolf, D-78465 Konstanz (DE); ZECH, Karl, D-78465 Konstanz (DE)
(86) Internationale Anmeldenummer: EP9401015
(87) Internationale Veröffentlichungsnummer: WO9422899

(56) Entgegenhaltungen:
- GB-A- 2 247 680
- US-A- 2 990 401

## Beschreibung

### Anwendungsgebiet der Erfindung

Die Erfindung betrifft neue Prednisolonderivate, die in der pharmazeutischen Industrie zur Herstellung von Medikamenten verwendet werden.

### Bekannter technischer Hintergrund

In der DE-OS 41 29 535 werden Pregna-1,4-dien-3,20-dion-16,17-acetal-21-ester offenbart, die am cyclischen Acetalring einen Butyl-, Isopropyl-, sec.-Butyl-, Cyclohexyl- oder Phenylrest tragen, und deren [-21-Hydroxylgruppe durch einen Acetyl- oder Isobutyrylrest acyliert ist.

### Beschreibung der Erfindung

Es wurde nun gefunden, daß die nachfolgenden erfindungsgemäßen Verbindungen, die sich von den Verbindungen der DE-OS 41 29 535 durch den fehlenden Acylrest an der C-21-Hydroxylgruppe unterscheiden, überraschende und vorteilhafte Eigenschaften besitzen.

Gegenstand der Erfindung sind die Epimeren der Verbindung der Formel I (siehe beiliegendes Formelblatt) in reiner Form sowie Gemische dieser Epimeren in jedem beliebigen Mischungsverhältnis.

Die Epimeren der Verbindung der Formel I können durch die Formeln Ia und Ib (siehe beiliegendes Formelblatt) charakterisiert werden.

Das Verfahren zur Herstellung der erfindungsgemäßen Verbindungen erfolgt dadurch, daß man 16-Hydroxyprednisolon mit Cyclohexanaldehyd umsetzt.

Die Umsetzung erfolgt auf eine dem Fachmann an sich bekannte Weise in geeigneten Lösungsmitteln wie Ethern, z.B. Dioxan, Diisopropylether, Estern, z.B. Essigsäureethylester, halogenierten Kohlenwasserstoffen, z.B. Methylenchlorid, Chloroform, nitrierten Kohlenwasserstoffen, z.B. Nitromethan, oder ohne Lösungsmittel, unter Zusatz von katalytischen oder auch größeren Mengen Säure, wie Mineralsäuren, z.B. Perchlorsäure, Chlorwasserstoffsäure, Tetrafluorborsäure, oder Sulfonsäuren, z.B. Methansulfonsäure, bei Temperaturen von vorzugsweise 0 bis 60°C. Bevorzugt wird die Umsetzung zum Epimerengemisch (Formel I) in Dioxan oder Essigsäureethylester mit 70 %iger Perchlorsäure oder 85 %iger Tetrafluorborsäure bei 0°C bis Raumtemperatur durchgeführt.

Die Umsetzung von 16-Hydroxyprednisolon mit Cyclohexanaldehyd liefert in der Regel ein Epimerengemisch, wobei durch geeignete Variation der Reaktionsbedingungen die Umsetzung so gesteuert werden kann, daß überwiegend ein bestimmtes Epimer entsteht.

Zur überwiegenden Herstellung des R-Epimeren (Formel Ia) werden beispielsweise folgende Bedingungen bevorzugt: Halogenierte Kohlenwasserstoffe oder Nitromethan mit Methansulfonsäure bei Raumtemperatur bis 40°C, oder 35-70 %ige Perchlorsäure bei 0°C bis Raumtemperatur. Eine weitere Möglichkeit zur überwiegenden Herstellung des R-Epimeren besteht in der Behandlung des Epimerengemisches (Formel I) mit 70 %iger Perchlorsäure in einem geeigneten Lösungsmittel, wie z.B. Methylenchlorid, bei 0°C (Epimerisierung).

Die überwiegende Herstellung des S-Epimeren (Formel Ib) wird mit Hilfe von Chlorwasserstoffgas in einem Lösungsmittel wie beispielsweise Dioxan bei 0°C bis Raumtemperatur erreicht.

Sofern ein Epimer in reinerer Form gewünscht wird, als dies aufgrund der Reaktionsbedingungen erzielbar ist, können der Umsetzung geeignete Trenn- und Reinigungsschritte, wie beispielsweise präparative HPLC, nachgeschaltet werden.

Die folgenden Beispiele dienen der näheren Erläuterung der Erfindung:

### Beispiele

**1.** 500 mg (1,3 mmol) 16-Hydroxyprednisolon werden in 5 ml Nitromethan suspendiert und mit 33 µl (0,38 mmol) 70 %iger Perchlorsäure und 195 µl (1,6 mmol) Cyclohexanaldehyd versetzt. Nach 4,5 h Rühren bei Raumtemperatur (Epimerenverhältnis in der Reaktionsmischung R/S = 55/45, HPLC-Gehalt 95 %) wird die Reaktionsmischung mit Natriumhydrogencarbonatlösung versetzt, der Niederschlag abgesaugt, mit Wasser und Nitromethan gewaschen und bei 50°C im Hochvakuum getrocknet. Ausbeute: 440 mg (70 %), Epimerenverhältnis R/S = 57/43 (bestimmt über HPLC, stationäre Phase ODS Hypersil, mobile Phase Wasser/Ethanol = 60/40).

**2.** 2,0 g (5,3 mmol) 16-Hydroxyprednisolon werden in 20 ml Nitromethan suspendiert und mit 0,88 ml (10,2 mmol) 70 %iger Perchlorsäure und 0,78 ml (6,4 mmol) Cyclohexanaldehyd versetzt. Nach 5 h Rühren bei Raumtemperatur (Epimerenverhältnis in der Reaktionsmischung R/S = 73/27, HPLC-Gehalt 95 %) wird wie in Beispiel I aufgearbeitet. Ausbeute: 1,96 g (78 %), Epimerenverhältnis R/S = 76/24.

**3.** 2,0 g (5,3 mmol) 16-Hydroxyprednisolon werden in 10 ml Nitromethan suspendiert und 1,5 ml (17,4 mmol) 70 %ige Perchlorsäure und anschließend 0,8 ml (6,6 mmol) Cyclohexanaldehyd zugetropft. Man rührt 2 h bei Raumtemperatur (Epimerenverhältnis in der Reaktionsmischung R/S = 92/8, HPLC-Gehalt 98 %) und arbeitet auf wie in Beispiel 1. Ausbeute: 2,2 g (88 %), Epimerenverhältnis R/S = 92/8.

**4.** 2,0 g (5,3 mmol) 16-Hydroxyprednisolon werden in 20 ml Nitromethan suspendiert und mit 3,52 ml (41 mmol) 70 %iger Perchlorsäure und 0,78 ml (6,4 mmol) Cyclohexanaldehyd versetzt. Nach 1 h Rühren bei Raumtemperatur wird auf Natriumhydrogencarbonatlösung gegeben, mit Methylenchlorid extrahiert, die organische Phase mit Natriumsulfat getrocknet und im Vakuum eingeengt. Der Rückstand wird auf Kieselgel mit Methylenchlorid/Essigsäureethylester = 1/1 chromatographiert (R_{f}=0,5). Ausbeute: 1,0 g (40 %), Epimerenverhältnis R/S = 89/11.

**5.** 20 g (53 mmol) 16-Hydroxyprednisolon werden in 300 ml Chloroform suspendiert, mit 8,0 ml (66 mmol) Cyclohexanaldehyd versetzt und unter Kühlung im Eisbad 17,6 ml (205 mmol) 70 %ige Perchlorsäure zugetropft. Nach 2,5 h Rühren bei Raumtemperatur wird die Reaktionsmischung auf Sodalösung gegeben, die organische Phase mit Wasser extrahiert, mit Natriumsulfat getrocknet und im Vakuum eingeengt (Epimerenverhältnis im Rohprodukt R/S = 85/15). Der Rückstand wird in warmem Ethanol gelöst, bis zur Trübung mit Wasser versetzt, im Eisbad gekühlt und der Niederschlag abgesaugt und getrocknet. Ausbeute: 20,2 g (81 %), Epimerenverhältnis R/S = 85/15.

**6.** 5,0 g (13,3 mmol) 16-Hydroxyprednisolon werden in 100 ml Methylenchlorid suspendiert, mit 4,4 ml (51,2 mmol) 70 %iger Perchlorsäure versetzt und 2,1 ml (17,3 mmol) Cyclohexanaldehyd zugetropft. Nach 1,25 h wird das Reaktionsgemisch auf Sodalösung gegeben, die organische Phase mit Wasser gewaschen, mit Magnesiumsulfat getrocknet und im Vakuum eingeengt. Rohausbeute quantitativ, HPLC-Gehalt 96 %, Epimerenverhältnis R/S = 89/11.

**7.** 300 g (797 mmol) 16-Hydroxyprednisolon werden in 3.0 l Essigsäureethylester suspendiert, mit 120 ml (991 mmol) Cyclohexanaldehyd versetzt und innerhalb von 20 Min. 150 ml (1,75 mol) 70 %ige Perchlorsäure zugetropft. Nach 1 h Rühren wird die Lösung mit 250 g Natriumcarbonat versetzt und mit 1,5 l Wasser ausgerührt. Die Wasserphase wird mit Essigsäureethylester, die gesammelten organischen Phasen werden mit gesättigter Natriumchloridlösung extrahiert. Nach Trocknen der organischen Phase mit Natriumsulfat wird im Vakuum langsam eingeengt, der entstehende Feststoff abgesaugt, mit Diethylether nachgewaschen und getrocknet. Ausbeute: 282 g (75 %), Epimerenverhältnis R/S = 58/42.

**8.** 10,0 g (26,6 mmol) 16-Hydroxyprednisolon werden unter Kühlung im Eisbad in 100 ml Dioxan suspendiert, mit 8,8 ml (102,4 mmol) 70 %iger Perchlorsäure versetzt und innerhalb von 45 Min. 3,7 ml (30,5 mmol) Cyclohexanaldehyd zugetropft. Man rührt 2 h bei Raumtemperatur, neutralisiert mit Sodalösung und extrahiert mit Methylenchlorid. Die organische Phase wird mit Wasser gewaschen, mit Natriumsulfat getrocknet und im Vakuum eingeengt (Epimerenverhältnis im Rohprodukt R/S 49/51). Der Rückstand wird in warmem Ethanol aufgenommen und durch Zugabe von Wasser und Kühlung im Eisbad fraktionierend kristallisiert. 1. Fraktion: 8,5 g, Epimerenverhältnis R/S 60/40. 2. Fraktion: 2,5 g, Epimerenverhältnis R/S = 27/73. Gesamtausbeute: 11 g (88 %).

**9.** 0,5 g (1,3 mmol) 16-Hydroxyprednisolon werden bei Raumtemperatur in 20 ml Diisopropylether suspendiert und mit 190 µl (1,56 mmol) Cyclohexanaldehyd und 440 µl (5,1 mmol) 70 %iger Perchlorsäure versetzt. Nach 45 Min. wird die Reaktionsmischung mit Essigsäureethylester versetzt und mit Natriumhydrogencarbonatlösung und Wasser extrahiert. Die organische Phase wird mit Magnesiumsulfat getrocknet und im Vakuum eingeengt. Rohausbeute quantitativ; HPLC-Gehalt 95 %, Epimerenverhältnis R/S = 57/43.

**10.** 2,0 g (5,3 mmol) 16-Hydroxyprednisolon werden bei Raumtemperatur in 20 ml Nitromethan suspendiert und mit 1,4 ml (21,5 mmol) Methansulfonsäure und 0,78 ml (6,4 mmol) Cyclohexanaldehyd versetzt. Die Lösung wird 3 h bei 40°C gerührt und nach Abkühlen mit Methylenchlorid verdünnt. Die Reaktionsmischung wird mit Natriumhydrogencarbonatlösung und Wasser extrahiert, die organische Phase mit Natriumsulfat getrocknet und im Vakuum eingeengt. Der Rückstand wird wie in Beispiel 4 chromatographiert. Ausbeute: 1,7 g (68 %), Epimerenverhältnis R/S = 85/15.

**11.** 5,0 g (13,3 mmol) 16-Hydroxyprednisolon werden in 50 ml Methylenchlorid suspendiert, unter Kühlung im Eisbad mit 3,45 ml (53,1 mmol) Methansulfonsäure versetzt und innerhalb von 10 Min. 1,95 ml (16,1 mmol) Cyclohexanaldehyd zugetropft. Man läßt auf Raumtemperatur kommen und rührt dann 3 h bei 40°C. Die Lösung wird mit Wasser extrahiert, mit Natriumsulfat getrocknet und im Vakuum eingeengt. Rohprodukt quantitativ, HPLC-Gehalt 96 %, Epimerenverhältnis R/S = 85/15.

**12.** 10,0 g (26,6 mmol) 16-Hydroxyprednisolon werden unter Kühlung im Eisbad in 60 ml 70 %iger Perchlorsäure suspendiert und innerhalb von 10 Min. mit 3,7 ml (30,5 mmol) Cyclohexanaldehyd versetzt. Nach 30-minütigem Rühren unter Eiskühlung wird auf eisgekühlte Natriumhydrogencarbonatlösung gegeben und mit Essigsäureethylester extrahiert. Die organische Phase wird mit Natriumhydrogencarbonatlösung und Wasser gewaschen, mit Natriumsulfat getrocknet und im Vakuum eingeengt (Epimerenverhältnis im Rohprodukt R/S = 93/7). Der Rückstand wird wie in Beispiel 8 gereinigt. 1. Fraktion: 2,1 g, Epimerenverhältnis R/S = 94,5/5,5, 2. Fraktion: 6,56 g, Epimerenverhältnis R/S = 96/4, 3. Fraktion: 1,29 g, Epimerenverhältnis R/S = 91,5/8,5. Gesamtausbeute: 9,95 g (79,5 %).

Bei entsprechender Umsetzung der Edukte in 50 bzw. 35 %iger Perchlorsäure wird im Rohprodukt ein Epimerenverhältnis von R/S = 95/5 bzw. 81/19 erhalten.

**13.** 5,0 g (13,3 mmol) 16-Hydroxyprednisolon werden unter Kühlung im Eisbad in 80 ml Dioxan suspendiert, mit 2,5 ml 85 %iger Tetrafluorborsäure in Diethylether versetzt und innerhalb von 10 Min. 1,95 ml (16,1 mmol) Cyclohexanaldehyd zugegeben. Man rührt 1 h bei Raumtemperatur, gießt dann auf Natriumhydrogencarbonatlösung und extrahiert mit Essigsäureethylester. Die organische Phase wird mit Wasser extrahiert, mit Natriumsulfat getrocknet und im Vakuum eingeengt. Der Rückstand wird wie in Beispiel 4 chromatographiert. Ausbeute: 4,0 g (64 %), Epimerenverhältnis R/S = 47/53.

**14.** 100 mg (0,27 mmol) 16-Hydroxyprednisolon werden in 5 ml Nitromethan suspendiert, mit 50 µl 85 %iger Tetrafluorborsäure in Diethylether und 35 µl (0,29 mmol) Cyclohexanaldehyd versetzt und 15 h bei Raumtemperatur gerührt. Epimerenverhältnis der Reaktionsmischung R/S = 80/20, HPLC-Gehalt 96 %. Die Einstellung des Epimerenverhältnisses kann auch durch 30 Min. Erwärmen der Reaktionslösung auf 60°C erreicht werden.

**15.** 2,0 g (5,3 mmol) 16-Hydroxyprednisolon werden in 40 ml Dioxan suspendiert, unter Kühlung im Eisbad mit 760 µl (6,3 mmol) Cyclohexanaldehyd versetzt und innerhalb von 20 Min. 15 ml 14,8 %ige Chlorwasserstoffgas/Dioxanlösung zugetropft. Nach 2 h Rühren bei 0°C und 2 h bei Raumtemperatur wird auf Natriumhydrogencarbonatlösung gegeben und mit Essigsäureethylester extrahiert. Die organische Phase wird mit Wasser gewaschen, mit Natriumsulfat getrocknet und im Vakuum eingeengt. Der Rückstand wird wie in Beispiel 4 chromatographiert. Ausbeute: 620 mg (25 %), Epimerenverhältnis R/S = 25/75.

**16.** 12,0 g (25,5 mmol) 16α,17-Cyclohexylmethylendioxy-11β,21-dihydroxypregna-1,4-dien-3,20-dion (Verbindung I, Epimerenverhältnis R/S = 60/40) werden bei 0°C in 240 ml Methylenchlorid gelöst, mit 8,7 ml (101,1 mmol) 70 %iger Perchlorsäure versetzt und nach 40 Min. Rühren auf Natriumhydrogencarbonatlösung gegeben. Man extrahiert die Wasserphase mit Methylenchlorid, die gesammelten organischen Phasen mit Wasser und trocknet mit Magnesiumsulfat. Nach Einengen des Lösungsmittels im Vakuum wird die Verbindung I quantitativ zurückgewonnen mit einem Epimerenverhältnis von R/S = 90/10 (HPLC-Gehalt 98 %).

**17.** Eine Trennung der Epimeren (ausgehend von einem beliebigen Epimerengemisch) kann mit Hilfe von HPLC beispielsweise wie folgt erzielt werden:

Geräte: HP 10848 Liquid Chromatograph, HP 79850B LC Terminal und UV-Detektor; Säulenmaterial: Hypersil C18, 12 µm, 250x20 mm; Eluent: Wasser (59 %) / Ethanol (41 %); Detektorwellenlänge: 242 nm; Probenkonzentration: 220 mg in 600 µl DMSO + 3800 µl Ethanol; Aufgabevolumen: 200 µl = 10 mg Epimerengemisch; Fluß: 10 ml/min; Ofentemperatur: 40°C; erreichte Reinheit: R-Epimer 99,6 %, S-Epimer 99,4 %.

### Gewerbliche Anwendbarkeit

Die erfindungsgemäßen Verbindungen besitzen wertvolle pharmakologische Eigenschaften, die sie gewerblich verwertbar machen. Sie sind generell für die Behandlung solcher Krankheitszustände geeignet, die durch steroidale Antiphlogistika therapiert werden können. Hierzu zählen in erster Linie Erkrankungen der Haut sowie des Respirationstraktes, aber auch entzündliche Darmerkrankungen sowie allergische Rhinitis/Conjunctivitis.

Im Bereich der Haut eignen sich die erfindungsgemäßen Verbindungen aufgrund ihrer antiphlogistischen, antiproliferativen, immunsuppressiven, antipruriginösen und vasokonstriktorischen Eigenschaften zur (insbesondere topischen) Behandlung von Dermatosen verschiedener Genese. Beispielsweise seien genannt: Allergisches Kontaktekzem, atopisches Ekzem, seborrhoisches Ekzem, Lichen simplex, Psoriasis (vulgaris), Sonnenbrand, Pruritus im Genitoanalbereich, Alopecia areata, hypertrophe Narben und diskoider Lupus erythematodes.

Im Bereich des Respirationstraktes unterdrücken die erfindungsgemäßen Verbindungen nahezu sämtliche in der Wand der Atemwege ablaufende Entzündungsreaktionen, indem sie die Proliferation, Differenzierung, Migration und Aktivierung der Entzündungszellen sowie die Bildung von Prostaglandinen, Leukotrienen und PAF hemmen. Hierdurch verringern die erfindungsgemäßen Verbindungen die bronchiale Hyperreaktivität, vermindern die Schleimbildung, verbessern die mukoziliäre Clearance und verstärken (z.T. durch vermehrte Expression von β-Adrenozeptoren) die Wirkung von β-Sympathomimetika. Aufgrund dieser Eigenschaften eignen sich die erfindungsgemäßen Verbindungen in erster Linie (in inhalativer Form topisch appliziert) für die (Langzeit)therapie des Asthma bronchiale.

Die erfindungsgemäßen Verbindungen zeichnen sich durch eine geringe Toxizität, eine im wesentlichen topische Wirksamkeit, eine große therapeutische Breite, eine langanhaltende Wirkung und das Fehlen wesentlicher Nebenwirkungen aus. Die Wirksamkeit der erfindungsgemäßen Verbindungen ermöglicht ihren Einsatz in der Human- und Veterinärmedizin.

Ein weiterer Gegenstand der Erfindung ist daher ein Verfahren zur Behandlung von Säugetieren einschließlich Menschen, die an einer der oben genannten Krankheiten erkrankt sind. Das Verfahren ist dadurch gekennzeichnet, daß man dem erkrankten Säugetier eine therapeutisch wirksame und pharmakologisch verträgliche Menge einer oder mehrerer der erfindungsgemäßen Verbindungen verabreicht.

Weiterer Gegenstand der Erfindung sind die erfindungsgemäßen Verbindungen zur Anwendung bei der Behandlung und/oder Prophylaxe der genannten Krankheiten.

Ebenso betrifft die Erfindung die Verwendung der erfindungsgemäßen Verbindungen zur Herstellung von Arzneimitteln, die zur Behandlung und/oder Prophylaxe der genannten Krankheiten eingesetzt werden.

Weiterhin sind Arzneimittel zur Behandlung und/oder Prophylaxe der genannten Krankheiten, die eine oder mehrere der erfindungsgemäßen Verbindungen enthalten, Gegenstand der Erfindung.

Für die Behandlung von Dermatosen erfolgt die Anwendung der erfindungsgemäßen Verbindungen insbesondere in Form solcher Arzneimittel, die für eine topische Applikation geeignet sind. Für die Herstellung der Arzneimittel werden die erfindungsgemäßen Verbindungen (= Wirkstoffe) vorzugsweise mit geeigneten pharmazeutischen Hilfsstoffen vermischt und zu geeigneten Arzneiformulierungen weiterverarbeitet. Als geeignete Arzneiformulierungen seien beispielsweise Puder, Emulsionen, Suspensionen, Sprays, Öle, Salben, Fettsalben, Cremes, Pasten, Gele oder Lösungen genannt.

Welche Hilfsstoffe für die gewünschten Arzneiformulierungen geeignet sind, ist dem Fachmann aufgrund seines Fachwissens geläufig. Neben Lösemitteln, Gelbildnern, Salbengrundlagen und anderen Wirkstoffträgern können beispielsweise Antioxidantien, Dispergiermittel, Emulgatoren, Konservierungsmittel, Lösungsvermittler oder Permeationspromotoren verwendet werden.

Für die Behandlung von Erkrankungen des Respirationstraktes werden die erfindungsgemäßen Verbindungen bevorzugt inhalativ appliziert. Hierzu werden diese entweder direkt als Pulver (vorzugsweise in mikronisierter Form) oder durch Vernebeln von Lösungen oder Suspensionen, die sie enthalten, verabreicht. Bezüglich der Zubereitungen und Darreichungsformen wird beispielsweise auf die Ausführungen im Europäischen Patent 163 965 verwiesen.

Die erfindungsgemäßen Arzneimittel werden nach an sich bekannten Verfahren hergestellt. Die Dosierung der Wirkstoffe erfolgt in der für stark wirksame Glucocorticoide üblichen Größenordnung. So enthalten topische Applikationsformen (wie z.B. Salben) für die Behandlung von Dermatosen die Wirkstoffe in einer Konzentration von beispielsweise 0,1-1 %. Die Dosis für die inhalative Applikation beträgt üblicherweise zwischen 0,2 und 2 mg pro Tag. Die übliche (Erhaltungs-)Dosis bei systemischer Therapie liegt bei etwa 10 mg pro Tag, wobei im Fall schwerer Asthmaanfälle sowie insbesondere beim Status asthmaticus auch wesentlich höhere Dosen (z.B. 250-500 mg i.v.) zur Anwendung gelangen können.

### Pharmakologie

Versuchsdurchführung zur Erfassung der lokalen und systemischen Wirkung der zu testenden Verbindungen auf die Granulationsgewebsbildung nach Watte-Implantation bei der Ratte (Cotton-pellet-Methode):

Männliche Sprague-Dawley-Ratten (jeweils 8-16 Tiere je Dosis; Gewicht je Tier: 180-230 g) erhalten in Isofluran-Narkose und unter sterilen Bedingungen im Schulterblattbereich beidseitig je 1 Wattekügelchen (Hersteller: Firma Hartmann/Heidenheim; Wattekügelchen Gr. 2, Nr. 4865/2) von 13,0 ± 0,5 mg subcutan implantiert. Vor Versuchsbeginn werden in die für die Implantation in die linke Körperseite vorgesehenen Wattekügelchen jeweils alkoholische Lösungen (0,05 ml/Pellet; 96 %-iger Alkohol) der zu prüfenden Verbindungen instilliert. Zum Zeitpunkt der Implantation sind die Pellets trocken, d.h. die Substanzen haben sich auf den Wattefäden niedergeschlagen. Die Pellets der rechten Körperseite werden unbehandelt implantiert.

Im Verlauf von 7 Tagen bilden sich aufgrund des Fremdkörperreizes Granulome. Diese werden am 8. Tag aus den getöteten Tieren vorsichtig, d.h. unter Schonung der Bindegewebskapsel, exstirpiert, getrocknet (15 h bei 120°C) und gewogen. Durch Abzug des Gewichtsanteils der Wattekügelchen erhält man die Menge des neugebildeten Granulationsgewebes.

Als Maß für die antiproliferative Wirkung einer Verbindung dient die prozentuale Minderung des mittleren Granulom-Trockengewichtes einer behandelten Gruppe gegenüber der Kontrollgruppe (= 100 %).

An den linken Granulomen erfaßt man die lokale, an den rechten die systemische Wirkung einer Verbindung.

Zur Erfassung der systemischen Corticoidwirkung wurden auch die Frischgewichte von Thymus und Nebenniere bestimmt.

**Tabelle**

| A**ntiproliferative Wirkung der erfindungsgemäßen Verbindungen nach lokaler Verabreichung im chronischen Entzündungsmodell - gemessen am Einfluß auf die Granulationsgewebsbildung nach sc. Watte-Implantation (sog. Cotton-pellet-Test) -** | | | | |
|---|---|---|---|---|
| Verbindung | Dosis 1 x (mg je Tier) lokal* | Hemmung der Granulationsgewebsbildung | | n (Zahl der Tiere) |
| | | % | p (Signifikanz) | |
| Ia | 0,2 | 69 | < 0,001 | 8 |
| Ib | 0,2 | 32 | < 0,001 | 8 |

| | | | | |
|---|---|---|---|---|
| * instilliert ins linke Pellet | | | | |

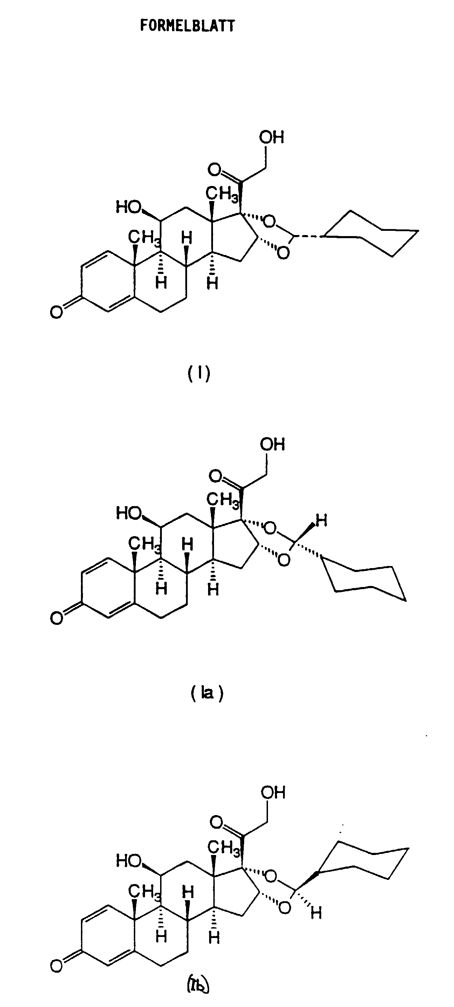

## Patentansprüche

1. Verbindung der Formel I 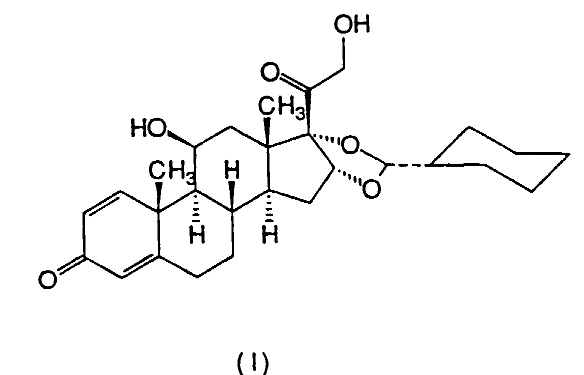 mit der chemischen Bezeichnung 16α,17-(22R,S)-Cyclohexylmethylendioxy-11β,21-dihydroxypregna-1,4-dien-3,20-dion.

2. Verbindung der Formel Ia 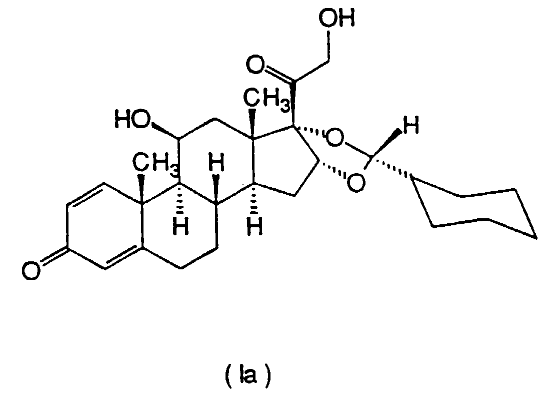 mit der chemischen Bezeichnung 16α,17-(22R)-Cyclohexylmethylendioxy-11β,21-dihydroxypregna-1,4-dien-3,20-dion.

3. Verbindung der Formel Ib 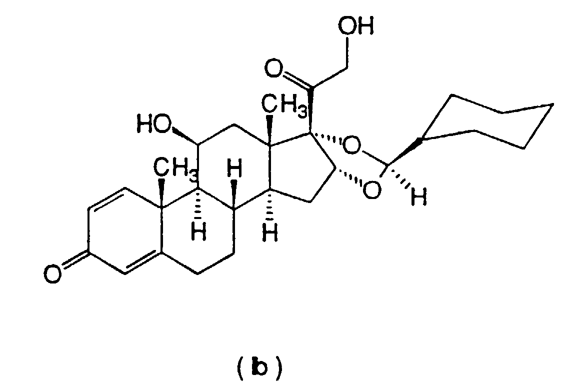 mit der chemischen Bezeichnung 16α,17-(22S)-Cyclohexylmethylendioxy-11β,21-dihydroxypregna-1,4-dien-3,20-dion.

4. Verbindung der Formel I nach Anspruch 1, dadurch gekennzeichnet, daß die Formel I für ein Epimerengemisch der Verbindungen Ia 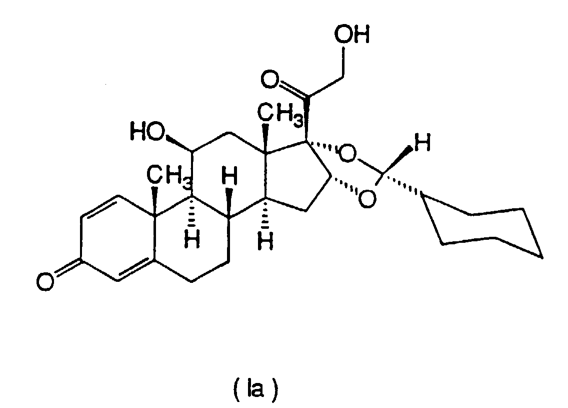 und Ib 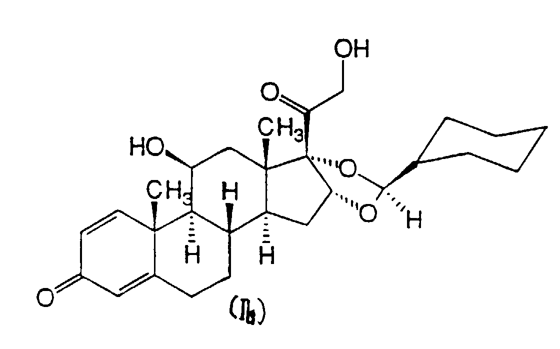 in jedem beliebigen Mischungsverhältnis steht.

5. Arzneimittel enthaltend eine Verbindung der Formel I nach Anspruch 1 als Epimerengemisch der Verbindungen Ia und Ib nach Anspruch 2 und 3 in jedem beliebigen Mischungsverhältnis.

6. Arzneimittel enthaltend eine Verbindung der Formel Ia nach Anspruch 2.

7. Arzneimittel enthaltend eine Verbindung der Formel Ib nach Anspruch 3.

8. Verbindung nach einem der Ansprüche 1 oder 2 oder 3 zur Anwendung bei der Behandlung und/oder Prophylaxe von Erkrankungen der Haut, Erkrankungen des Respirationstraktes, entzündlichen Erkrankungen des Darmes sowie der allergischen Rhinitis/Conjunctivitis.

9. Verwendung einer Verbindung nach einem der Ansprüche 1 oder 2 oder 3 zur Herstellung von Arzneimitteln zur Behandlung und/oder Prophylaxe von Erkrankungen der Haut, Erkrankungen des Respirationstraktes, entzündlichen Erkrankungen des Darmes sowie der allergischen Rhinitis/Conjunctivitis.

## Claims

1. A compound of the formula I 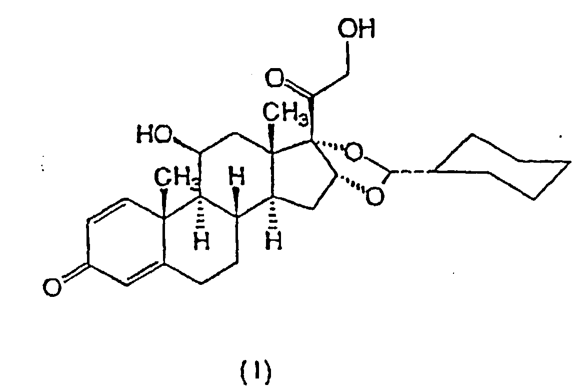 having the chemical name 16α,17-(22R,S)-cyclohexylmethylenedioxy-11β,21-dihydroxypregna-1,4-diene-3,20-dione.

2. Compound of the formula Ia 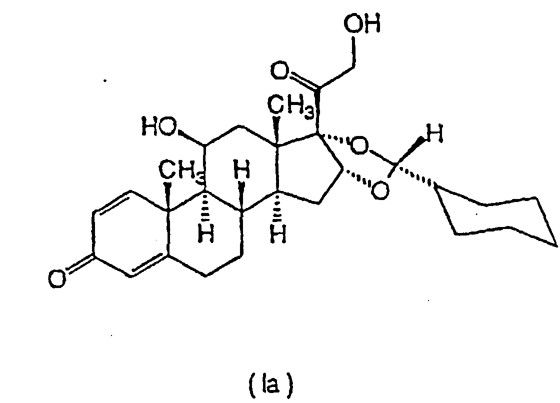 having the chemical name 16α,17-(22R)-cyclohexylmethylenedioxy-11β,21-dihydroxypregna-1,4-diene-3,20-dione.

3. A compound of the formula Ib 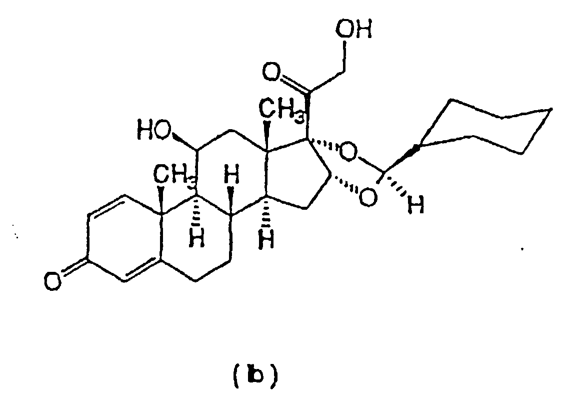 having the chemical name 16α,17-(22S)-cyclohexylmethylenedioxy-11β,21-dihydroxypregna-1,4-diene-3,20-dione.

4. A compound of the formula I as claimed in claim 1, wherein the formula I is an epimer mixture of the compounds Ia 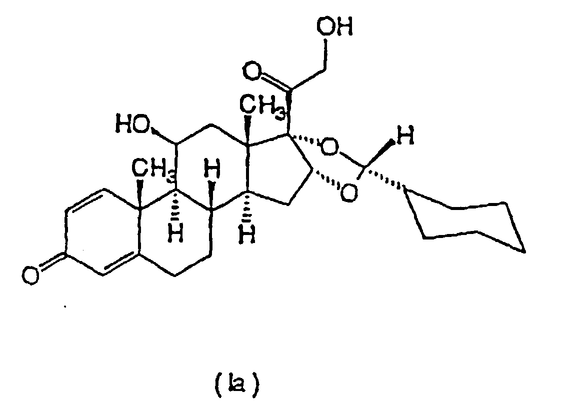 and Ib 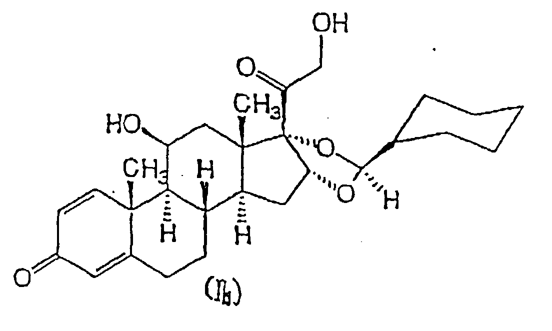 in any desired mixture ratio.

5. A medicament containing a compound of the formula I as claimed in claim 1 as an epimer mixture of the compounds Ia and Ib as claimed in claims 2 and 3 in any desired mixture ratio.

6. A medicament containing a compound of the formula Ia as claimed in claim 2.

7. A medicament containing a compound of the formula Ib as claimed in claim 3.

8. A compound as claimed in one of claims 1 or 2 or 3 for use in the treatment and/or prophylaxis of disorders of the skin, disorders of the respiratory tract, inflammatory disorders of the bowels, and of allergic rhinitis/conjunctivitis.

9. The use of a compound as claimed in one of claims 1 or 2 or 3 for the production of medicaments for the treatment and/or prophylaxis of disorders of the skin, disorders of the respiratory tract, inflammatory disorders of the bowels, and of allergic rhinitis/conjunctivitis.

## Revendications

1. Composé de formule (I) 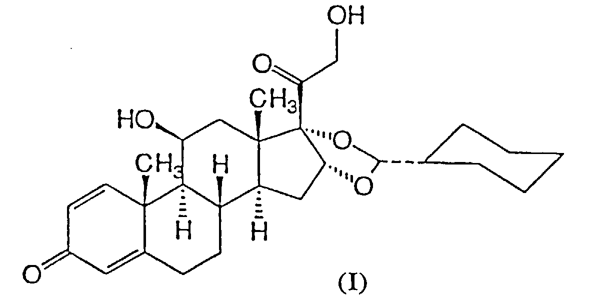 qui porte la dénomination chimique 16α,17-(22R,S)-cyclohexylméthylénedioxy-11β,21-dihydroxyprégnane-1,4-diène-3,3(1-dione.

2. Composé de formule (Ia) 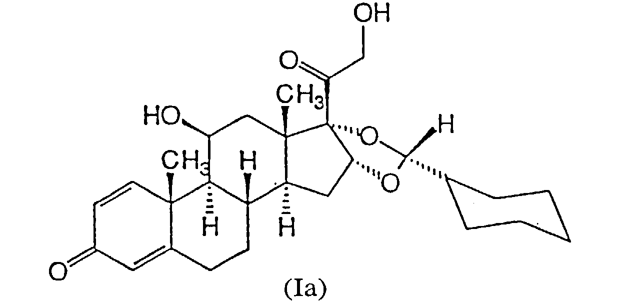 qui porte la dénomination chimique 16α,17-(22R)-cyclohexylméthylènedioxy-11β,21-dihydroxyprégnane-1,4-diène-3,30-dione.

3. Composé de formule (Ib) 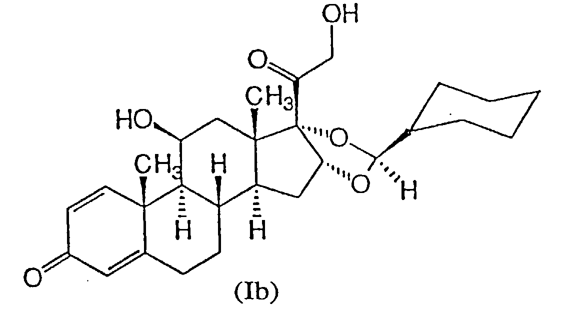 qui porte la dénomination chimique 16α,17-(22S)-cyclohexylméthylènedioxy-11β,21-dihydroxyprégnane-1,4-diène-3,30-dione.

4. Composé de formule (I) selon la revendication 1, caractérisé en ce que la formule (I) représente un mélange des épimères (Ia) et (Ib) suivants : 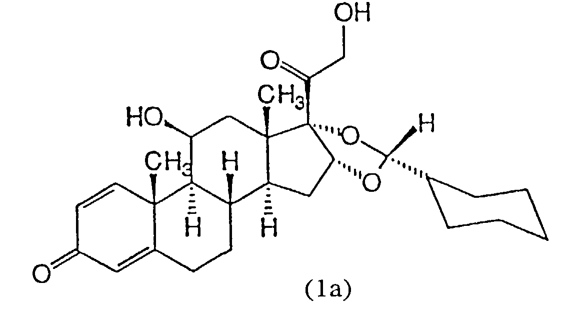 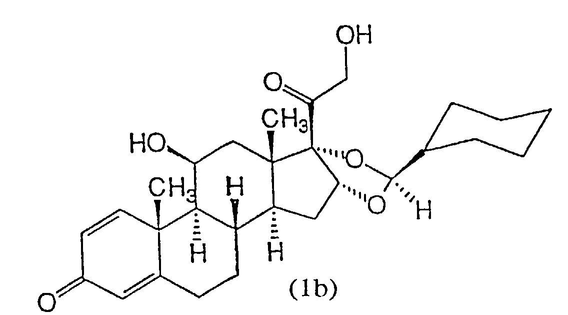 en n'importe quel rapport de mélange.

5. Médicament contenant un composé de formule (I) selon la revendication 1 sous forme de mélange d'épimères de formule (Ia) et (Ib) selon les revendications 2 et 3 en n'importe quel rapport de mélange.

6. Médicament contenant un composé de formule (Ia) selon la revendication 2.

7. Médicament contenant un composé de formule (Ib) selon la revendication 3.

8. Composé selon une des revendications 1 ou 2 ou 3 destiné à l'application pour le traitement thérapeutique et/ou prophylactique de maladies de la peau, de maladies du tractus respiratoire, de maladies inflammatoires de l'intestin ainsi que de rhinites/conjonctivites allergiques.

9. Utilisation d'un composé selon l'une des revendications 1 ou 2 ou 3 pour la préparation de médicaments destinés au traitement thérapeutique et/ou prophylactique de maladies de la peau, de maladies du tractus respiratoire, de maladies inflammatoires de l'intestin ainsi que de rhinites/conjonctivites allergiques.
